Europäisches Patentamt

European Patent Office

Office européen des brevets

(19)

(11) Publication number: **0 003 879**
**B1**

(12) **EUROPEAN PATENT SPECIFICATION**

(45) Date of publication of patent specification: **09.09.81**

(21) Application number: **79300196.7**

(22) Date of filing: **09.02.79**

(51) Int. Cl.³: **C 02 F 1/32,** C 02 F 1/78,
A 61 L 2/10

(54) **Apparatus for sanitising liquids.**

(30) Priority: **21.02.78 US 879204**

(43) Date of publication of application:
**05.09.79 Bulletin 79/18**

(45) Publication of the grant of the European patent:
**09.09.81 Bulletin 81/36**

(84) Designated Contracting States:
**BE DE FR GB NL SE**

(56) References cited:
**DE - A - 2 225 984**
**DE - A - 2 307 877**
**DE - A - 2 551 622**
**US - A - 3 336 099**

(73) Proprietor: **THETFORD CORPORATION**
**7101 Jackson Road**
**Ann Arbor Michigan 48103 (US)**

(72) Inventor: **Coviello, Allan J.**
**645 Hidden Valley Drive 102 Ann Arbor**
**Michigan 48104 (US)**
Inventor: **Bernardin, Frederick E., Jr.**
**2314 W 17th Street**
**Wilmington Delaware 19806 (US)**
Inventor: **Rohrkemper, Robert R.**
**263 Glazier**
**Chelsea Michigan 48118 (US)**

(74) Representative: **Barlow, Roy James et al,**
**J.A.KEMP & CO. 14, South Square Gray's Inn**
**London WC1R 5EU (GB)**

Courier Press, Leamington Spa, England.

## Apparatus for sanitising liquids

The present invention relates to apparatus for sanitising liquids comprising an elongate ultraviolet lamp, a duct surrounding the lamp, means to pass air through an axially extending zone of said duct at a predetermined velocity sufficient so that said air is irradiated and ozone is formed at a relatively high concentration, and means for contacting a liquid to be sanitised with air irradiated in said duct.

In the treatment of water for example upon purification for drinking purposes or upon treatment of waste water prior to discharge into the environment, it is conventional practice to disinfect the water for elimination of potentially harmful micro-organisms. It has long been conventional practice to utilise chlorine in liquid or gaseous form as the biocide to accomplish this task. Recent investigations have pointed out the potential disadvantages of chlorination treatment due to formation of harmful byproducts. Interest has increased, therefore, in the use of other biocides such as ultraviolet radiation and ozone.

It is well known that ultraviolet radiation at a wavelength of 2537 Å is an efficient germicide This is described in United States Patent No. 3,336,099. It is also known that the output of a lamp designed to produce 2537 Å wavelength is optimum at a particular operating temperature. Temperatures either warmer or cooler than the optimum will produce lower output of radiation. Further, it is also known that when air is subjected to ultraviolet radiation, at a wavelength of 1875 Å, ozone is produced from oxygen contained in the air.

As is disclosed in said United States Patent No. 3,336,099, waste water and air can be treated simultaneously by ultraviolet radiation in apparatus having a single lamp, and to further purify the water the treated water can then be contacted in a storage tank by the ozone that is produced. However, the construction and arrangement of the apparatus disclosed in said United States Patent 3,336,099 fails to achieve simultaneously both high efficiency of ultraviolet radiation to eliminate harmful micro-organisms and also optimum ozone production potential from the ultraviolet radiation source. In the disclosure of said United States Patent it is contemplated that the air to be irradiated should be cooled after passing through an air compressor, but it follows that when the cooled air is then passed in irradiating contact with the lamp, to achieve adequate cooling it must flow at a sufficiently rapid rate so that if the temperature of the lamp is maintained in a range wherein desired radiation efficiency is achieved, the ozone concentration of the air is relatively low. Thus, high radiation efficiency and optimum ozone concentration cannot be achieved simultaneously.

The present invention aims to overcome the inadequacies of the prior art of the type disclosed in United States Patent No. 3,336,099, and provides an improved apparatus in which the efficiency of ultraviolet radiation can be increased while simultaneously the ozone concentration potential from the ultraviolet radiation source can be optimised.

The present invention is characterised by means to pass cooling air along at least a portion of the length of the lamp at a velocity higher than said first velocity to cool the lamp to a temperature near that which will enable maximum radiation output of the lamp to be achieved. The air space or zone where the air is drawn along the lamp at a predetermined velocity allows the desired or optimum rate to be used for allowing sufficient contact time for a significant quantity of the oxygen contained in the air to be converted to ozone. The ozone-rich air can then be used for contacting the water to be treated. Also, the means for passing a higher velocity flow of air in contact with a portion of the lamp, allows adequate cooling of the lamp for its efficiency to be increased over that expected from a lamp operating uncooled.

In certain forms of the present invention, the apparatus includes passageways providing for separate paths of the cooling air and of the air to be efficiently irradiated for the production of ozone. For example, in one preferred form of the invention there may be a single inlet for the air from which a major portion is diverted for contact at a relatively high velocity with at least one end of the UV lamp, and the remainder of the air is drawn at a lower rate along the length of the lamp for ozone production. In other forms of the invention, the inlets for cooling air and irradiation air may be separate with separate passageways extending through the apparatus such that one passageway serves to cool only the end portions of the lamp and the other passageway provides for passage of air at a substantially slower velocity along the length of the lamp for the production of the ozone. In another form of the invention, a plurality of concentric passageways may be provided, with the inner one serving as a cooling passage through which air can travel rapidly, and the outer passageway serving to permit the flow of air at a relatively slow rate for the production of ozone.

In still another form of the invention, two-stage air contact is provided wherein the air initially travels at high velocity over the one end of the UV lamp because only a small restricted passageway is provided, and the second stage is defined by a relatively larger passageway through which the same air can then flow at a relatively slower velocity to provide production of the ozone.

A detailed understanding of this invention will result from reading the following description in which reference is made to the accompanying drawings, which form a part of this specification, and in which:-

Figure 1 is a vertical cross-section through one form of apparatus embodying the present invention, wherein separation of the passage of cooling air and irradiated air is provided;

Figure 2 is a similar illustration of a second embodiment of the invention, wherein separation of cooling air and irradiated air is provided;

Figure 3 is a similar illustration of still another embodiment of the invention, which also provides separate passageways for flow of cooling air and irradiated air;

Figure 4 illustrates in a similar view still another embodiment of the present invention wherein a single passageway for air is provided, and wherein two contact stages are provided, one for cooling purposes and the other for irradiation purposes;

Figure 5 illustrates still another embodiment of the invention, similar to that of Figure 3, but wherein a plurality of ultraviolet lamps is provided;

Figure 6 is a section taken on the lines VI—VI of Figure 5; and

Figure 7 is a schematic illustration showing the embodiment of Figure 1 in association with a storage tank for carrying out the treatment of the waste water.

It is to be understood that the invention is not limited in its application to all the details of construction, and arrangement of parts, which are illustrated in the accompanying drawings, purely for the purpose of exemplification. The invention is capable of other embodiments and of being practised or carried out in various ways within the scope of the claims of this specification.

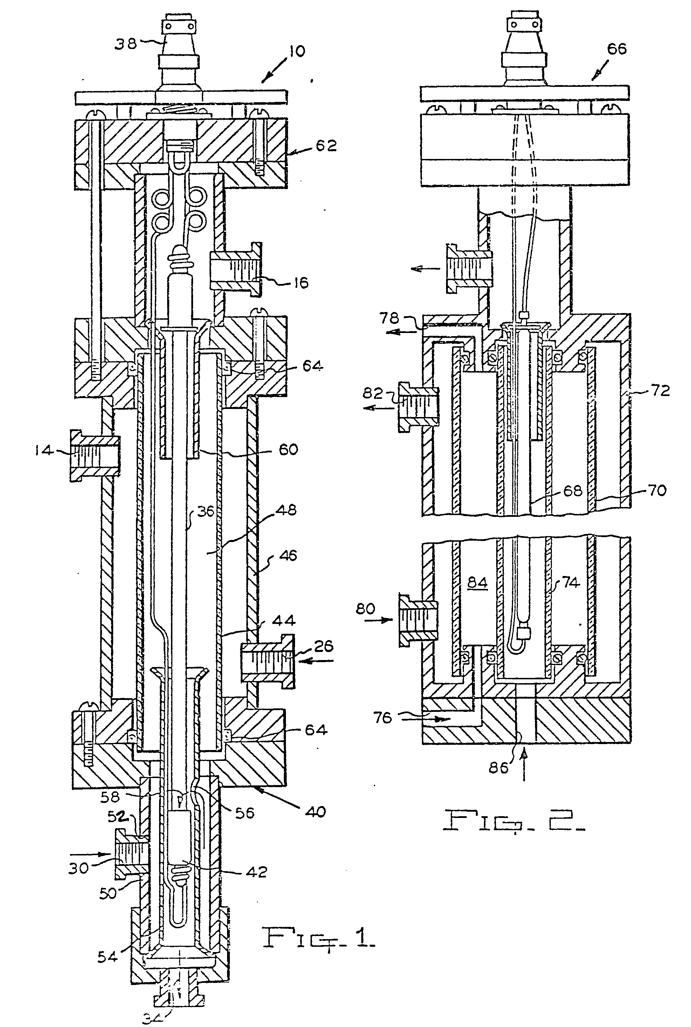

Referring now to the drawings, it will be seen that Figure 7 shows an overall apparatus 10, which can be any of the embodiments shown in Figures 1 to 5, but in this instance is the embodiment of Figure 1. Apparatus 10 is illustrated in association with water storage tank 12 into which the waste water is discharged along the conduit 14 after it has been contacted by ultraviolet light in the apparatus 10, and also into which tank has been discharged the irradiated air containing ozone which has been produced in apparatus 10. The irradiated air and ozone are transferred to the tank 12 via the conduit 16, by means of air pump 18 giving a preselected rate of flow. The irradiated air and ozone are contacted with the water in the tank 12 by means of the air diffuser 20, located adjacent to the bottom of the tank 12. The diffused air 22 passes upward from the air diffuser 20 through the waste water 24 which has been treated previously by exposure to the ultraviolet light. Thus, the ultraviolet light functions to carry out primary disinfection and the ozone in the air functions to carry out secondary disinfection of the waste water.

The waste water is initially delivered to the apparatus 10 via the conduit 26, and any suitable pump 28 can be used to move the waste water at a desired rate of flow through the apparatus 10 for discharge via the conduit 14 into the tank 12. A single source of air is provided, via the conduit 30, and any suitable pump or blower 32 can be employed to supply this air. As will be explained subsequently, the cooling air is discharged from the apparatus 10, as indicated by the arrow 34 in Figure 7, and a lesser quantity of air, which is to be irradiated, will pass through the apparatus 10 at the rate set by the pump 18 for discharge into the tank 12 as previously described.

Details of the apparatus 10, embodying a preferred form of the invention, will now be described with reference to Figure 1. The apparatus 10 includes a conventional elongate ultraviolet lamp or tube 36 and has an electrical connector fitting 38 at its upper end. A suitable ultraviolet lamp for this purpose is a standard G-37-T6 ultraviolet lamp.

A bottom end cap assembly 40 encloses the lower end 42 of the ultraviolet lamp 36 and supports the coaxial inner and outer ducts 44 and 46 thereabove. The inner duct is transparent, and is made of a suitable material, such as quartz, which is transparent to the germicidal wavelengths of the ultraviolet light. The outer duct 46 can be made of any suitable material, such as organic plastics or stainless steel, which are not readily susceptible to corrosion caused by the waste water.

Means, including the inlet conduit 30, are provided to pass air through the inner duct 44 so that the air is irradiated and ozone is formed primarily in the zone 48 defined between the ends of the inner duct 44 and the inner diameter of the duct 44. However, a major portion of the air entering at conduit 30 is used only for cooling the ultraviolet lamp 36 and will enter the lower outer sleeve 50 of the bottom cap assembly 40 by way of the inlet port 52 and will then flow around a guide wall (the lower inner sleeve 54) and through the port 56 generally in the direction of the arrow 58 for discharge out of the open lower end of the lower outer sleeve 50, thereby impinging directly upon the lower end of the ultraviolet lamp 36 to cool it. The lower inner sleeve 54 is connected to the lower outer sleeve 50 at the bottom thereof so that the annular duct formed between these two sleeves is closed at the bottom with the result that all of the air discharged at 34 must have impinged upon ultraviolet lamp 36.

Thus, the major portion of the air which enters via the conduit 30 will discharge at 34 through the lower end of the bottom cap assembly 40, with the minor portion of the air passing upward around the ultraviolet lamp 36 to be discharged via the conduit 16. As can be seen in Figure 1, the air passage available around the lower end 42 of the lamp 36 is relatively small so that the air which discharges at the lower end can flow at a relatively high velocity along the lamp.

The zone 48 which surrounds the middle portion of the lamp 36 has a relatively large inner diameter so that the air which passes through this zone can flow at a relatively slow velocity governed by the volumetric flow rate at which the pump 18 moves the air through this zone. When the irradiated air with the generated ozone leaves the zone 48, it will pass inside the inner upper sleeve 60 to the conduit 16.

As previously indicated, the waste water is introduced into the apparatus 10 *via* inlet conduit 26 to flow upward through the passageway defined between the duct 44 and the duct 46 for discharge *via* the conduit 14. When passing upward through the apparatus 10, the waste water will be irradiated by the ultraviolet light from lamp 36. The top cap assembly 62 can be any conventional construction for holding the ultraviolet lamp 36 and also to provide means for gaining access to the ducts 44 and 46, for example for cleaning purposes. To provide proper seals at the upper and lower ends of the ducts 44 and 46, conventional O-rings 64 are employed.

In operation of the preferred embodiment shown in Figure 1, it has been found that very satisfactory results are achieved utilising cooling air which enters apparatus 10 at approximately 27°C and which is discharged at the lower end of the bottom cap assembly at a rate of approximately 28 liters per minute while at the same time allowing air to flow through the zone 48 at a rate of from 2·5 litres per minute to 14 litres per minute. Ozone concentrations of from 0·498 to 0·115 milligrams per litre in the air were achieved. Thus, the slowest rate is preferred so that nearly 0·5 milligrams per litre can be obtained. Contacting the lower end of the ultraviolet lamp 36 with cooling air, as specified, resulted in the ultraviolet lamp 36 operating at a relatively high efficiency. This was carried out in conjunction with a lamp approximately one metre in length. The quartz sleeve or inner duct 44 had an internal diameter of 40 millimetres. Thus, it can be seen that with respect to the relative flow rates of air discharged at conduits 16 and 34, the rate is very substantially greater at 34 than at 16.

Preferably, the rate is at least ten times greater at 34 than at 16 under the described conditions of operation.

With respect to the rate of flow of the waste water through the outer duct defined by the sleeve 46, it is known that different organisms require different lethal dosages of ultraviolet radiation, for example, *Esherichia coli* requires 7040 micro watt-seconds per $cm^2$, while infectious hepatitis virus requires 8000 micro watt-seconds per $cm^2$. It is therefore desirable that the preferred embodiment, whose duct defined by sleeve 46 is of 1·6 litres volume, be operated at less than 62 litres per minute input and better still, in order to allow an adequate factor of safety, at less than 6·2 litres per minute.

Referring next to Figures 2, 3 and 4, other embodiments of the invention will be described, with particular reference to the passageways provided for the flow of the waste water and the air.

Figure 2 illustrates an alternative design of the apparatus, here referenced 66, which includes a conventional ultraviolet lamp 68, and an inner duct 70 surrounding the lamp 68 and formed from a material which is transparent to the germicidal wavelengths of the ultraviolet light from the lamp 68. An outer duct 72 surrounds the inner duct 70. This embodiment also includes a separate cooling duct 74 inside and isolated from air irradiating inner duct 70 and directly surrounding the ultraviolet lamp 68, and the wall of 74 is formed of quartz or similar material which is transparent both to the germicidal wavelengths of the ultraviolet light and to the wavelengths of the ultraviolet light for producing ozone from oxygen contained in air. A conduit 76, which also may include a pump (not shown), is provided for passing air through the inner or air-irradiating duct 70 so that the air in that duct 70 is irradiated, and ozone is formed fom the oxygen therein, and is discharged along conduit 78 for passage to the tank 12. Conduit 80, which may include a pump (not shown), is provided for passing waste water through the outer duct 72 so that the liquid can be irradiated and then discharged along conduit 82 connected to the tank 12. In this form of the invention, an air irradiating zone 84 of relatively large dimension is provided so that the air passing through the inner duct 70 can move at a slow rate to produce a relatively high concentration of ozone.

Cooling air conduit 86, which can also include a pump (not shown), is provided to pass cooling air along the length of the lamp 68 at a relatively fast rate to maintain the lamp 68 at a temperature sufficient to achieve a relatively high radiation output.

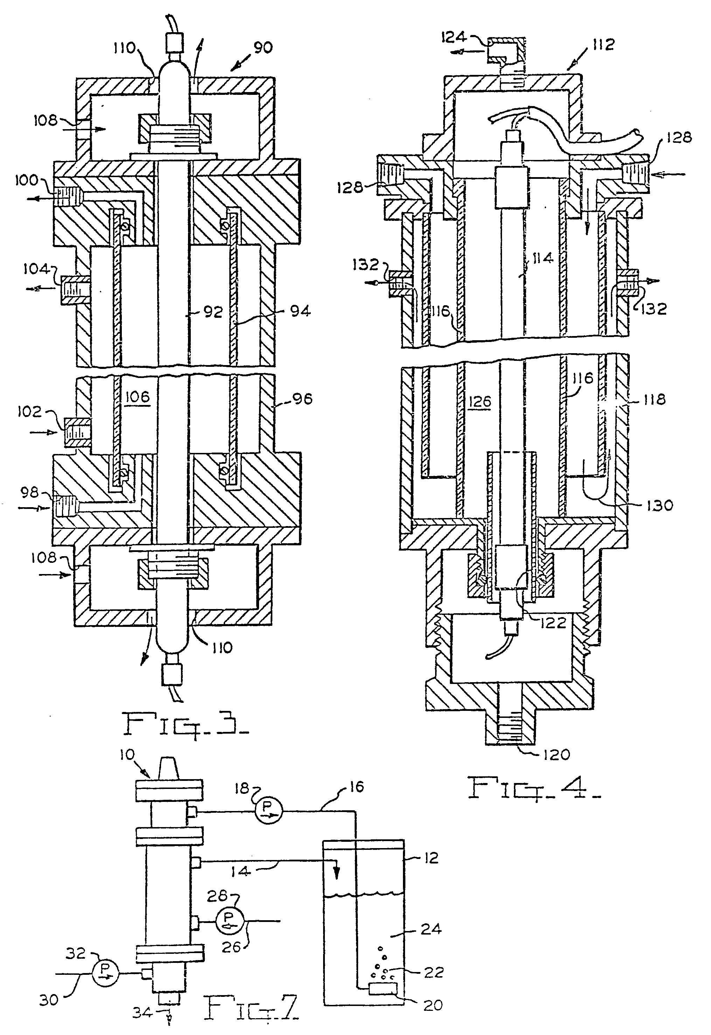

The alternative embodiment of apparatus 90, illustrated in Figure 3, includes a conventional elongated ultraviolet lamp 92, an inner duct 94 surrounding the lamp 92 and formed from a material which is transparent to the germicidal wavelengths of the ultraviolet light from lamp 92, and an outer duct 96 surrounding the inner duct 94. Air conduit 98, which may include a pump (not shown), is provided for passing air through the inner duct 94 so that the air is irradiated and ozone is formed. The irradiated air containing the ozone is then discharged at 100 to the tank 12. The water inlet conduit 102, which may include a pump (not shown), is provided to pass waste water through the outer duct 96 so that the liquid is irradiated and can be discharged along conduit 104 which is connected to the tank 12. In this form of the apparatus the inner duct 94 includes a zone 106 wherein the air will flow at a predetermined relatively slow rate so that, when irradiated, it will produce a relatively high concentration of ozone. Also, this embodiment of the invention includes respective cooling air conduits 108, at the upper and lower ends of apparatus 90, to pass cooling air along end portions of the length of the lamp 92 at a relatively high rate to maintain the lamp 92 at a temperature sufficient to achieve relatively high radiation output. This air can then be discharged at conduits 110. Cooling air pumps of suitable form (not shown), may be provided.

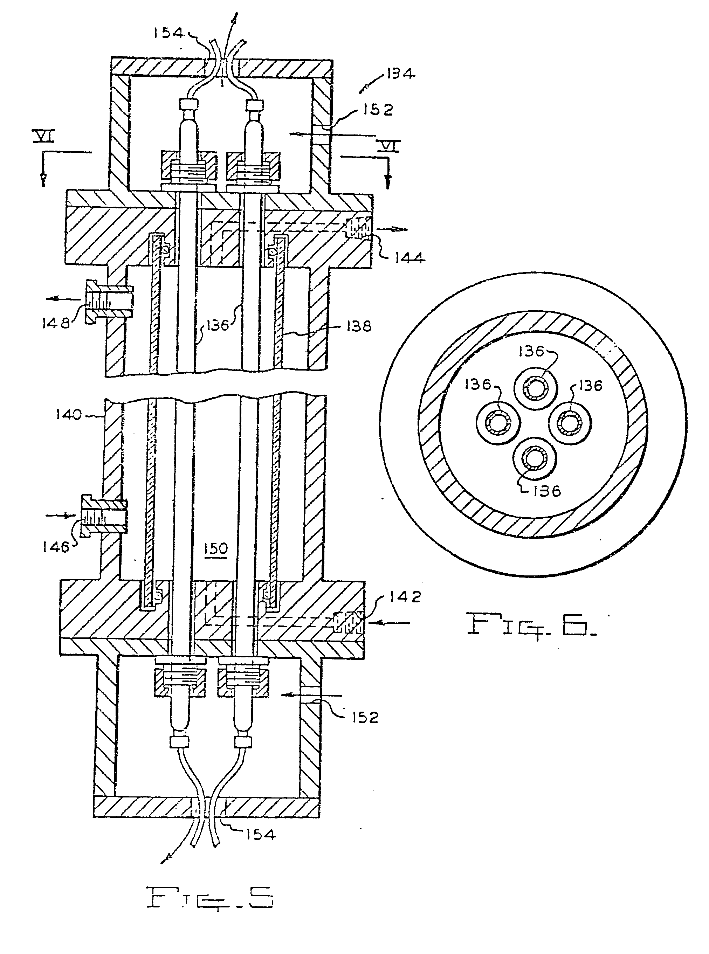

In the embodiment of the apparatus illustrated in Figure 4, the apparatus 112 includes a conventional ultraviolet tube or elongate lamp 114, an inner duct 116 surrounding the lamp 114 and formed from a material which is transparent to the germicidal wavelengths of the ultraviolet light from lamp 114, and an outer duct 118 surrounding the inner duct 116. An air conduit 120, which may include a pump (not shown), is provided to pass air first through an inner sleeve 122, which sleeve may be made of a suitable material such as quartz, and then through the inner duct 116 for discharge along conduit 124 connected to the tank 12. This air will be irradiated while passing through the enlarged zone 126 and ozone will be formed. A conduit 128, which may include a pump (not shown), is provided to pass liquid through the outer duct 118 along the path indicated by arrows 130 to be discharged along conduits 132 connected to the tank 12. During its passage, this water will be irradiated by the ultraviolet light emitted from lamp 114. The water thus first passes downwardly in contact with the wall of inner duct 116 in a first irradiation pass and is then deflected upwardly around the outside of a transparent intermediate guide sleeve to undergo a second irradiating step before discharging through conduits 132.

Also, in this embodiment the air irradiating zone 126 is substantially larger in diameter or effective cross section than the cooling air passage provided between the sleeve 122 and the lamp 114 so that the air entering along conduit 120 will, in a first state of operation, flow rapidly in contact with at least a portion of the lamp 114 after which it will flow at a relatively slow rate through the zone 126. Passage through the first stage will effect cooling of the lamp 114 and passage through the second stage will effect optimum production of ozone by irradiation.

The embodiment of the apparatus 134 shown in Figure 5 includes a plurality of conventional elongate ultraviolet lamps 136, in parallel relationship to the longitudinal axis of and adjacent to a surrounding inner air irradiation duct 138 formed from a material which is transparent to the germicidal wavelengths of the ultraviolet light from lamps 136. An outer duct 140 surrounds the inner duct 138. An air conduit 142, which may include a pump (not shown), is provided for passing air through the inner duct 138 so that the air is irradiated and ozone is formed. The irradiated air containing the ozone is then discharged along conduit 144 to the tank 12. A water conduit 146, which may include a pump (not shown), is provided to pass waste water through the outer duct 140 where it is irradiated before being discharged along conduit 148 connected to the tank 12. In this form of the apparatus of the invention, the inner duct 138 includes a zone 150 wherein the air will flow at a predetermined relatively slow rate so that, when irradiated, it will produce a relatively high concentration of ozone. Also, this embodiment of the invention includes cooling air conduits 152 at the upper and lower ends of the apparatus 134 to pass cooling air along portions of the length of the lamps 136 at a relatively high rate to maintain the lamps 136 at a temperature, in the electrode region of each lamp, low enough to achieve relatively high radiation output. This air can then be discharged at 154. A cooling air pump (not shown) may be included, if desired.

This form of the invention has the advantage that a large air-irradiation zone 150 can be utilised to provide a relatively high concentration of ozone, and also that the lamps are in close proximity to the waste water flowing in the outer duct 140 serving a two-fold purpose of making maximum use of the germicidal wavelengths of the lamps 136 and also of using the cooling effect on the lamps that can be derived from the nearby flow of the waste water in a heat-exchange relationship.

From the foregoing description it will be evident that in all of the embodiments described, air is introduced into the apparatus 10, 66, 90, 112 and 134 to produce optimum cooling of the ultraviolet lamp or lamps therein by movement of the air at a relatively high velocity along at least a portion of the length of the lamp, and air is also moved at a relatively slow rate through a zone exposed to the ultraviolet light so that optimum production of ozone is realised. Simultaneously, the waste water is treated with the same ultraviolet light, and the rate of flow of the waste water is set so that optimum germicidal treatment can occur.

As suggested earlier herein, the apparatus of this invention can be used as either pre- or posttreatment means for water handling equipment, for example for purifying drinking water or for treating waste water before discharge.

## Claims

1. Apparatus for sanitising liquids comprising an elongate ultraviolet lamp, a duct surrounding the lamp, means to pass air through an axially extending zone of said duct at a predetermined velocity sufficient so that said air is irradiated and ozone is formed at a relatively high concentration, means for contacting a liquid to be sanitised with air irradiated in said duct, characterised by means (54), (86), (108,110), (122),

(152,154) to pass cooling air along at least a portion of the length of the lamp at a velocity higher than said first velocity to cool the lamp to a temperature near that which will enable maximum radiation output of the lamp to be achieved.

2. Apparatus according to claim 1, characterised in that said means to pass cooling air along at least a portion of the length of said lamp comprises a high velocity flow stage portion (54 or 60) (122) of said duct (48,54,60) (122,126) where said duct is a multi-stage contacting duct with at least one high velocity small diameter contact stage portion (54,60) (122) and a larger diameter lower velocity contact stage portion (48) (126).

3. Apparatus according to claim 2, characterised in that said larger diameter low velocity contact stage portion of the duct includes a relatively large diameter sleeve (44) (116) through which the air moves slowly exposed to said lamp to increase ozone concentration and said smaller diameter high velocity contact stage portion includes at least one relatively smaller diameter sleeve (54,60) (122) through which the air must flow at a higher velocity to promote cooling of the lamp.

4. Apparatus according to claim 1, characterised in that said means to pass cooling air along at least a portion of the length of the lamp includes a passage (108,110) (152,154) for the cooling air separate from said duct (106) (150); and in that said duct surrounds the midportion of said lamp (92)(136) inward from the ends of the lamp and provides passage for the irradiated air only along said midportion at a relatively slow rate, whereas said means to pass cooling air encloses at least one of the ends of said lamp and provides for passage of the cooling air at a relatively high velocity.

5. Apparatus according to claim 1, characterised in that there are separate passages (84) for the irradiated air and (74) for the cooling air, and said means to pass cooling air includes a cooling passage (74) having its wall formed of duct (84) and surrounding said lamp (68), said cooling paassage (74) having its wall formed of a material which is transparent to the wavelengths of the ultraviolet light for producing ozone from oxygen contained in air.

6. Apparatus according to claim 1, characterised in that said means to pass cooling air along at least a portion of the length of the lamp includes a common inlet port (30) for the air and separate passages for the irradiated air and the cooling air to separate outlet ports (16 and 34), and said means to pass cooling air includes a guide wall (54) for directing a portion of the incoming air through said duct (48) at a relatively slow rate and for directing the remainder (58) of the incoming air as a coolant over one end of the lamp (36) at a relatively higher velocity.

7. Apparatus according to claim 6, characterised in that said common inlet port (30) is formed in an outer sleeve (50) carried below a bottom cap assembly (40) of said apparatus; in that the lower end of said lamp extends into said outer sleeve (50); in that said guide wall comprises a lower inner sleeve (54) surrounding the lower end (42) of said lamp (36) and extending upward toward said duct (48); in that said lower inner sleeve (54) is spaced from said lower outer sleeve (50) and connected at the bottom thereto to provide an annular duct for air to pass upward from said common inlet port (30); in that said lower inner sleeve (54) also has a port (56) through which air can pass from said annular duct into contact with said lamp (36) and then to be discharged (at 34) through the bottom open end of said lower outer sleeve (50) so that a major portion of the air entering said lower outer sleeve can be removed rapidly through the bottom end for cooling the lower end of said lamp and the remainder can pass relatively slowly upward through said inner duct (48) to be irradiated.

8. Apparatus according to claim 1, or 4, characterised in that there is a plurality of ultraviolet lamps (136) surrounded by said duct (138), said lamps being arranged in parallel relationship to the longitudinal axis of said duct (138) and positioned radially outwardly of the axis to locations adjacent to the duct wall (138).

9. Apparatus according to any one of claims 1 to 8, further characterised in that the said contacting means comprise a tank connected to said duct and arranged to contact irradiated air from said duct with a said liquid to be sanitised.

10. Apparatus according to claim 9, characterised in that the wall of said duct (48) (84) (106) (126) (150) surrounding the lamp is formed from a material which is transparent to the germicidal wavelengths of ultraviolet light emitted by said lamp; and in that an outer duct (46) (72) (96) (118) (140) surrounds said transparent-walled duct (48) (84) (106) (126) said tank to pass a liquid to said tank (12) from said outer duct (46) (72) (96) (118) (140) where said liquid is irradiated; in that said transparent-walled duct (48) (8) (106) (126) (150) is connected to an outlet in said tank to pass the irradiated air into said tank for contacting the irradiated liquid from said outer duct.

11. Apparatus according to claim 9 or 10, characterised in that an air pump (18) is provided to pass the irradiated air into said tank, said air pump being operable to control the rate that the air flows through said duct to said tank.

12. Apparatus according to any one of the preceding claims, characterised in that in use of the apparatus air passes through said axially extending zone at a velocity of 1/10 or less of that at which said cooling air passes said portion of the length of the lamp.

**Patentansprüche**

1. Einrichtung zur Entkeimung von Flüssigkeiten. mit einer länglichen UV-Lampe. einem die UV-Lampe umschließenden Strömungskanal, einer Vorrichtung zum Hindurchleiten von Luft durch eine axial verlaufende Zone des Strömungskanals mit einer vorbestimmten und ausreichenden Strömungsgeschwindigkeit, um die Luft zu bestrahlen und Ozon in relativ hoher Konzentration zu erzeugen, und mit einer Vorrichtung zum Inkontaktbringen einer zu entkeimenden Flüssigkeit mit der in dem Strömungskanal bestrahlten Luft, gekennzeichnet durch eine Einrichtung (54; 86; 108, 110; 122; 152, 154), zur Führung von Kühlluft längs mindestens eines Teil der Länge der UV-Lampe mit einer über der vorgenannten Strömungsgeschwindigkeit liegenden Strömungsgeschwindigkeit, um dadurch eine Abkühlung der UV-Lampe zumindest annähernd auf eine die maximale Strahlungsleistung liefernde Temperatur zu erhalten.

2. Einrichtung nach Anspruch 1, dadurch gekennzeichnet, daß der Strömungskanal mehrere Kontaktstufen aufweist, wobei mindestens ein Abschnitt mit kleinerem Durchmesser eine Hockgeschwindigkeitskontaktstufe (54; 60; 122) und ein Abschnitt mit grösserem Durchmesser eine Kontaktstufe (48, 126) für niedrigere Strömungsgeschwindigkeit bildet, und daß die Einrichtung zur Führung von Kühlluft längs mindestens eines Teils der Länge der UV-Lampe einen Abschnitt (54 oder 60; 122) einer Hochgeschwindigkeits-Stufe des Strömungskanals (48, 54, 60; 122, 126) umfasst.

3. Einrichtung nach Anspruch 2, dadurch gekennzeichnet, daß die Kontaktstufe für niedrigere Strömungsgeschwindigkeit des Strömungskanals einen Mantel (44, 116) mit relativ grossem Durchmesser aufweist, durch den die der UV-Lampe ausgesetzte Luft zum Zweck der Erhöhung der Ozonkonzentration langsam strömt, und daß die Hochgeschwindigkeits-Kontaktstufe mindestens einen Mantel (54, 60; 122) mit relativ kleinerem Durchmesser aufweist, den die Luft zum Zweck der Kühlung der UV-Lampe mit höherer Geschwindigkeit durchströmen muß.

4. Einrichtung nach Anspruch 1, dadurch gekennzeichnet, daß die Einrichtung zur Führung von Kühlluft längs mindestens eines Teils der Länge der UV-Lampe für die Kühlluft einen von dem Strömungskanal (106, 150) getrennten Durchlaß (108, 110; 152, 154) aufweist, daß der Strömungskanal den Mittelteil der UV-Lampe (92, 136) zwischen den Enden der UV-Lampe umschließt und einen Durchlaß für eine relativ niedrige Strömungsgeschwindigkeit nur längs dieses Mittelbereiches bildet, und daß die Einrichtung zur Führung der Kühlluft mindestens eines der Enden der UV-Lampe umschließt und einen Durchlaß für die Kühlluft mit relativ hoher Strömungsgeschwindigkeit schafft.

5. Einrichtung nach Anspruch 1, dadurch gekennzeichnet, daß für die bestrahlte Luft und für die Kühlluft jeweils getrennte Durchlässe (84 bzw. 74) vorgesehen sind, daß der Durchlaß (74) für die Kühlluft in der Einrichtung zur Führung von Kühlluft innerhalb des Durchlasses (84) für die Bestrahlung angeordnet ist und die UV-Lampe (68) umgibt, und daß die Wandung des Durchlasses (74) für die Kühlluft aus einem Werkstoff besteht, der für die UV-Strahlung mit denjenigen Wellenlängen durchlässig ist, die zur Erzeugung von Ozon aus dem in der Luft enthaltenen Sauerstoff führt.

6. Einrichtung nach Anspruch 1, dadurch gekennzeichnet, daß die Einrichtung zur Führung von Kühlluft längs mindestens eines Teils der Länge der UV-Lampe eine gemeinsame Einlaßöffnung (30) für Luft und getrennte Durchlässe für bestrahlte Luft und Kühlluft aufweist, die zu getrennten Auslaßöffnungen (16 bzw. 34) führen, sowie eine Führungswand (54) besitzt, um einen Teil der eintretenden Luft durch den Strömungskanal (48) mit relativ niedriger Geschwindigkeit und den Rest (58) als Kühlmittel über ein Ende der UV-Lampe (36) mit relativ höherer Geschwindigkeit zu leiten.

7. Einrichtung nach Anspruch 6, dadurch gekennzeichnet, daß die gemeinsame Einlaßöffnung (30) in einem Außenmantel (50) unterhalb einer Bodenkappenanordnung (40) der Einrichtung ausgebildet ist, daß das untere Ende der UV-Lampe in den Außenmantel (50) hineinragt, daß die Führungswand mit einem unteren Innenmantel (54) das untere Ende (42) der UV-Lampe (36) umgibt und sich nach oben zu dem Strömungskanal (48) hin erstreckt, daß der untere Innenmantel (54) in einem Abstand von dem unteren Außenmantel (50) angeordnet und damit am unteren Ende derart verbunden ist, daß ein Ringkanal zur Führung von Luft von der gemeinsamen Einlaßöffnung (30) entsteht, und daß der untere Innenmantel (54) eine Offnung (56) aufweist, durch welche Luft aus dem Ringkanal in Kontakt mit der UV-Lampe (36) geführt und anschließend (bei 34) durch das bodenseitige offene Ende des unteren Außenmantels (50) freigegeben werden kann, so daß der Hauptteil der durch den unteren Außenmantel (50) eintretenden Luft rasch durch das bodenseitige offene Ende zum Zweck der Kühlung des unteren Lampenendes abgezogen werden kann, während der Rest relativ langsam nach oben durch den inneren Strömungskanal (48) zum Zweck der Bestrahlung strömt.

8. Einrichtung nach Anspruch 1 oder 4, dadurch gekennzeichnet, daß mehrere UV-Lampen (136) von dem Strömungskanal (138) umschlossen sind, die parallel zur Längsachse des Strömungskanals (138) angeordnet und radial außerhalb der Achse nahe der Wandung des Strömungskanals (138) positioniert sind.

9. Einrichtung nach einem der Ansprüche 1 bis 8, dadurch gekennzeichnet, daß die Vorrichtung zum Inkontaktbringen einer zu entkeimenden Flüssigkeit mit der in dem Strömungskanal bestrahlten Luft einen Tank umfasst, der an den Strömungskanal angeschlossen ist.

10. Einrichtung nach Anspruch 9, dadurch gekennzeichnet, daß die Wand des die UV-Lampe umgebenden Strömungskanals (48, 84, 106, 126, 150) aus einem Werkstoff besteht, der für die von der UV-Lampe abgegebene Strahlung mit keimtötender Wellenlänge durchlässig ist, daß der Strömungskanal von einer äußeren Flüssigkeitsführung (46, 72, 96, 118, 140) umgeben ist, die an den Tank (12) angeschlossen ist und in der zum Tank (12) geführte Flüssigkeit bestrahlt wird, und daß der Strömungskanal (48, 84, 106, 126, 150) an einen innerhalb des Tanks (12) gelegenen Auslaß angeschlossen ist, durch den bestrahlte Luft in den Tank (12) austritt und mit der bestrahlten Flüssigkeit aus der Flüssigkeitsführung in Kontakt gelangt.

11. Einrichtung nach Anspruch 9 oder 10, dadurch gekennzeichnet, daß eine Luftpumpe (18) zur Förderung der bestrahlten Luft in den Tank (12) vorgesehen ist, die zur Steuerung der Strömungsgeschwindigkeit der Luft durch den Strömungskanal zum Tank steuerbar ist.

12. Einrichtung nach einem der vorangehenden Ansprüche, dadurch gekennzeichnet, daß die axial verlaufende Zone des Strömungskanals derart ausgelegt isb, daß Luft im Betrieb mit einer Strömungsgeschwindigkeit von 1/10 oder darunter der Geschwindigkeit darin strömt, mit der die Kühlluft längs eines Teils der UV-Lampe strömt.

**Revendications**

1. Appareil pour purifier des liquides comprenant une lampe ultraviolette de forme allongée, un conduit entourant la lampe, des moyens pour faire passer de l'air à travers une zone dudit conduit s'étendant axialement, à une vitesse prédéterminée suffisante pour que ledit air soit irradié et que l'ozone soit formé à une concentration relativement élevée, et des moyens pour mettre en contact le liquide à purifier avec l'air irradié dans ledit tube, caractérisé par des moyens (54), (86), (108, 110), (122), (152, 154) pour faire passer de l'air de refroidissement le long d'au moins une partie de la longueur de la lampe à une vitesse supérieure à ladite première vitesse pour refroidir la lampe à une température voisine de celle qui permet une émission maximum de radiation à partir de la lampe.

2. Appareil selon la revendication 1, caractérisé par le fait que lesdits moyens pour faire passer l'air le long d'au moins une partie de la longueur de ladite lampe comprennent une partie d'étage à vitesse d'écoulement élevée (54 ou 60) (122) dudit conduit (48, 54, 60) (122, 126), ledit conduit étant un conduit de contact à plusieurs étages ayant au moins un étage de contact de faible diamètre à vitesse élevée (54, 60) (122) et un étage de contact de plus grand diamètre à plus faible vitesse (48) (126).

3. Appareil selon la revendication 2, caractérisé par le fait que l'étage de contact du conduit de plus grand diamètre et à faible vitesse comprend un manchon de diamètre relativement grand (44) (116) à travers lequel l'air exposé à ladite lampe se déplace lentement pour accroître la concentration en ozone et que ledit étage de contact de plus faible diamètre à vitesse élevée comprend au moins un manchon de diamètre relativement faible (54, 60) (122) à travers lequel l'air doit s'écouler à une vitesse élevée pour favoriser le refroidissement de la lampe.

4. Appareil selon la revendication 1, caractérisé par le fait que les moyens pour faire passer l'air de refroidissement le long d'au moins une partie de la longueur de la lampe comprennent un passage (108, 110) (152, 154) pour l'air de refroidissement, qui est séparé dudit conduit (106) (150); et que ledit conduit entoure la partie médiane de ladite lampe (92) (136) entre les extrémités de la lampe et constitue un passage pour l'air irradié uniquement le long de ladite partie médiane a un débit relativement faible, tandis que les moyens pour faire passer l'air de refroidissement entourent au moins l'une des extrémités de ladite lampe et permettent le passage de l'air de refroidissement à une vitesse relativement élevée.

5. Appareil selon la revendication 1, caractérisé par le fait qu'il existe des passages séparés (84) pour l'air irradié et (74) pour l'air de refroidissement et que les moyens précités pour faire passer l'air de refroidissement comprennent un passage de refroidissement (74) disposé à l'intérieur du conduit d'irradiation (84) précité et entourant ladite lampe (68), ledit passage de refroidissement (74) ayant sa paroi faite en un matériau transparent aux longueurs d'ondes de la lumière ultraviolette pour la production d'ozone à partir de l'oxygène contenue dans l'air.

6. Appareil selon la revendication 1, caractérisé par le fait que les moyens précités pour faire passer l'air de refroidissement le long d'au moins une partie de la lampe comprennent un orifice d'entrée commun (30) pour l'air et des passages séparés pour l'air irradié et l'air de refroidissement en direction d'orifices de sortie séparés (16 et 34) et que les moyens précités pour faire passer l'air de refroidissement comprennent une paroi de guidage (54) pour diriger une partie de l'air qui pénètre à l'intérieur dudit conduit (48) à un débit relativement faible et pour diriger la partie restante (58) de l'air qui y pénètre, en tant que fluide de refroidissement, sur une extrémité de la lampe (36) à une vitesse relativement élevée.

7. Appareil selon la revendication 6, caractérisé par le fait que l'orifice d'entrée commun (30) précité est formé dans un manchon extérieur (50) supporté par un chapeau inférieur (40) dudit appareil; que l'extrémité inférieure de la lampe s'étend à l'intérieur du manchon extérieur (50); que ladite paroi de guidage comprend un manchon inférieur (54), disposé à l'intérieur, entourant l'extrémité inférieure (42) de ladite lampe (36) et s'étendant vers le haut vers ledit tube (48); que le manchon inférieur (54) précité, disposé à l'intérieur, est écarté dudit manchon inférieur (50) périphérique et relié à la base de celui-ci pour constituer un conduit annulaire permettant à l'air de s'élever à partir dudit orifice d'entrée commun (30); que ledit manchon inférieur (54) disposé à l'intérieur, a également un orifice (56) à travers lequel l'air peut passer à partir dudit conduit annulaire et venir en contact avec la paroi de la lampe (36) pour être ensuite déchargé (en 34) à travers l'extrémité inférieure ouverte dudit manchon inférieur (50) disposé à l'extérieur, de sorte qu'une majeure partie de l'air pénétrant dans ledit manchon inférieur, disposé à l'extérieur, peut être éliminé rapidement à travers l'extrémité ouverte inférieure pour refroidir l'extrémité inférieure de ladite lamp, la partie restante de l'air pouvant passer à une vitesse relativement faible vers le haut, à travers ledit conduit intérieur (48) pour être irradiée.

8. Appareil selon l'une des revendications 1 ou 4, caractérisé par le fait qu'il est prévu une pluralité de lampes ultraviolettes (136) entourées par ledit conduit (138), lesdites lampes étant disposées parallèlement à l'axe longitudinal dudit conduit (138) et positionnées radialement à l'extérieur de l'axe, dans des zones adjacentes à la paroi du conduit (138).

9. Appareil selon l'une des revendications 1 à 8, caractérisé par le fait que les moyens de contact précités comprennent un réservoir relié audit conduit et disposé de façon à mettre en contact l'air irradié provenant dudit conduit avec le liquide à purifier.

10. Appareil selon la revendication 9, caractérisé par le fait que la paroi dudit conduit (48) (84) (106) (126) (150) entourant la lampe est faite en un matériau transparent aux longueurs d'ondes germicides de la lumière ultraviolette émise par ladite lampe; et qu'un conduit extérieur (46) (72) (96) (118) (140) entoure ledit conduit à paroi transparente et est relié audit réservoir pour faire passer un liquide dans ledit réservoir (12) à partir dudit tube extérieur (46) (72) (96) (118) (140) où ledit liquide est irradié; que ledit conduit à paroi transparente (48) (84) (106) (126) (150) est relié à une sortie pratiquée dans ledit réservoir pour faire passer l'air irradié dans ledit réservoir et le mettre en contact avec le liquide irradié provenant dudit conduit extérieur.

11. Appareil selon l'une des revendications 9 ou 10, caractérisé par le fait qu'il est prévu une pompe à air (18) pour faire passer l'air irradié dans ledit réservoir, ladite pompe à air pouvant être actionnée pour contrôler le débit avec lequel l'air s'écoule dans ledit réservoir en passant par ledit conduit.

12. Appareil selon l'une des revendications 1 à 11, caractérisé par le fait que, lors de l'utilisation dudit appareil, l'air passe à travers la zone précitée s'étendant axialement à une vitesse égale à 1/10ème ou moins, de celle à laquelle ledit air de refroidissement passe le long de ladite partie de la longueur de la lampe.

FIG. 1.

FIG. 2.

FIG_3.

FIG_4.

FIG_7

Fig. 5.

Fig. 6.